(19) Europäisches Patentamt
European Patent Office
Office européen des brevets

(11) Veröffentlichungsnummer: **0 220 254**
**B1**

(12) **EUROPÄISCHE PATENTSCHRIFT**

(45) Veröffentlichungstag der Patentschrift: **24.10.90**

(21) Anmeldenummer: **86902825.8**

(22) Anmeldetag: **19.04.86**

(86) Internationale Anmeldenummer:
**PCT/EP86/00235**

(87) Internationale Veröffentlichungsnummer:
**WO 86/06372 06.11.86 Gazette 86/24**

(51) Int. Cl.$^5$: **C 07 D 239/30,**
C 07 D 239/26,
C 07 D 237/08,
C 07 D 213/79,
C 07 D 213/55,
C 07 D 213/30, C 09 K 19/34

(54) **BENZONITRILE.**

(30) Priorität: **02.05.85 DE 3515633**

(43) Veröffentlichungstag der Anmeldung:
**06.05.87 Patentblatt 87/19**

(45) Bekanntmachung des Hinweises auf die Patenterteilung:
**24.10.90 Patentblatt 90/43**

(84) Benannte Vertragsstaaten:
**AT BE CH DE FR GB IT LI NL SE**

(56) Entgegenhaltungen:
**EP-A-0 025 119**
**EP-A-0 097 033**
**EP-A-0 199 004**
**WO-A-60/3769**
**JP-A-56 164 170**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **KRAUSE, Joachim**
**Samuel-Morse-Strasse 14**
**D-6110 Dieburg (DE)**
Erfinder: **POETSCH, Eike**
**Am Buchwald 4**
**D-6109 Mühltal 6 (DE)**
Erfinder: **HITTICH, Reinhard**
**Am Kirchberg 11**
**D-6101 Modautal 1 (DE)**
Erfinder: **SCHEUBLE, Bernhard**
**Am Grenzweg 18**
**D-6146 Alsbach (DE)**
Erfinder: **WEBER, Georg**
**Wilhelm-Leuschner-Strasse 38**
**D-6106 Erzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 220 254 B1

**Beschreibung**

Die Erfindung betrifft Benzonitrile der Formel I,

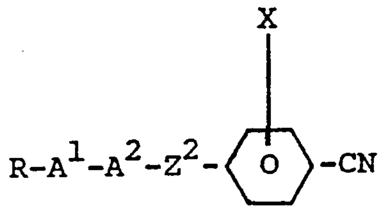

(I)

worin

R Alkyl mit 1—15 C-Atomen, worin auch eine oder zwei nicht benachbarte $CH_2$-Gruppen durch —O— ersetzt sein können, oder R'—$A^3$—$Z^3$—,

R' H, Alkyl mit 1—15 C-Atomen, worin auch eine oder zwei nicht benachbarte $CH_2$-Gruppen durch —O— ersetzt sein können,

$A^1$ Pyrimidin-2,5-diyl, Pyridin-2,5-diyl, Pyridazin-3,6-diyl oder 1,4-Phenylen,

$A^2$ Pyrimidin-2,5-diyl, Pyridin-2,5-diyl, 1,4-Phenylen oder 1,4-Cycohexylen,

$A^3$ Pyrimidin-2,5-diyl

$Z^2$ —CO—O—, —$CH_2CH_2$—, —$CH_2O$—,

$Z^3$ —CO—O—,

X F, Cl oder $CF_3$ bedeutet,

mit den Maßgaben, daß

a) mindestens einer der Ringe $A^1$, $A^2$, $A^3$ ein stickstoffhaltiger aromatischer Heterocyclus ist, und

b) trans-4-(5-Pentylpyrimidin-2-yl)-cyclohexylmethyl-(p-cyan-m-fluorphenyl)-ether,
trans-4-(5-Heptylpyrimidin-2-yl)-cyclohexylmethyl-(p-cyan-m-fluorphenyl)-ether,
trans-4-(5-Nonylpyrimidin-2-yl)-cyclohexylmethyl-(p-cyan-m-fluorphenyl)-ether,
trans-4-(5-Decylpyrimidin-2-yl)-cyclohexylmethyl-(p-cyan-m-fluorphenyl)-ether,
trans-4-(5-Dodecylpyrimidin-2-yl)-cyclohexylmethyl-(p-cyan-m-fluorphenyl)-ether,
p-(5-n-Heptylpyrimidin-2-yl)-benzyl-(4-cyan-3-fluorphenyl)-ether,
p-(5-Propylpyrimidin-2-yl)-benzyl-(4-cyan-3-fluorphenyl)-ether,
p-(5-Butylpyrimidin-2-yl)-benzyl-(4-cyan-3-fluorphenyl)-ether,
p-(5-Pentylpyrimidin-2-yl)-benzyl-(4-cyan-3-fluorphenyl)-ether,
p-(5-Hexylpyrimidin-2-yl)-benzyl-(4-cyan-3-fluorphenyl)-ether,
p-(5-Octylpyrimidin-2-yl)-benzyl-(4-cyan-3-fluorphenyl)-ether,
p-(5-Nonylpyrimidin-2-yl)-benzyl-(4-cyan-3-fluorphenyl)-ether,
p-(5-Decylpyrimidin-2-yl)-benzyl-(4-cyan-3-fluorphenyl)-ether und
p-(5-Dodecylpyrimidin-2-yl)-benzyl-(4-cyan-3-fluorphenyl)-ether,
ausgeschlossen sind.

Der Einfachheit halber bedeuten im folgenden Cy eine 1,4-Cyclohexylengruppe, Phe eine 1,4-Phenylengruppe, Phe- X, CN, eine in 2- oder 3-Stellung durch Fluor, Chlor oder Trifluormethyl substituierte 4-Cyanophenylgruppe, Pyd eine Pyridazin-3,6-diylgruppe, Pym eine Pyrimidin-2,5-diylgruppe und Pyre eine Pyridin-2,5-diylgruppe.

Die Verbindungen der Formel I können als Komponenten flüssigkristalliner Phasen verwendet werden, insbesondere für Displays, die auf dem Prinzip der verdtrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind und insbesondere vorteilhafte Werte für die optische und dielektrische Anisotropie aufweisen.

Aus der JP—A—56 164 170 sind ähnliche Verbindungen bekannt, die jedoch anstelle eines Cyano-Restes einen Alkylrest aufweisen und somit eine negative oder neutrale Anisotropie der Dielektrizitätskonstanten aufweisen.

Aus der EP—A—0 025 119 sind weiterhin (4-(5-Alkylpyrimidin-2-yl)-phenyl)-4-cyanobenzoesäureester bekannt, die aufgrund ihrer weniger positiven dielektrischen Anisotropie weniger für moderne Flüssigkristall-Displays geeignet sind als die erfindungsgemäßen.

Aus der EP—A—0 097 033 sind ähnliche Verbindungen mit 2 Ringgliedern bekannt, welche lediglich monotrope Mesophasenbereiche aufweisen.

In der EP—A—0 199 004, die einen Stand der Technik gemäß Artikel 54 (3) EPÜ präsentiert, sind einige (4-(5-Alkylpyrimidin-2-yl)-cyclohexylmethyl bzw. -benzyl)-(4-cyano-3-fluorphenyl)-ether genannt. Diese sind gemäß Maßgabe b) ausgenommen.

Aus der EP—0 019 665 sind Dicyanphenylester mit positiver dielektrischer Anisotropie bekannt, die einen unpolaren Cyclohexanring enthalten. Es wurde nun gefunden, daß Verbindungen der Formel I, die

einen polarisierbaren, stickstoffhaltigen aromatischen Heterocyclus und das polarisierende Strukturelement eines substituierten Benzonitrils enthalten, als Komponenten flüssigkristalliner Phasen vorzüglich geeignet sind. Insbesondere verfügen sie über besonders vorteilhafte Werte für die optische und dielektrische Anisotropie. Auch können mit ihrer Hilfe stabile flüssigkristalline Phasen mit breitem Mesophasenbereich und vergleichsweise niedriger Viskosität erhalten werden.

Weiterhin zeichnen sich die Verbindungen der Formel I durch besonders vorteilhafte elastische Konstanten aus.

Mit der Bereitstellung der Verbindungen der Formel I wird außerdem ganz allgemein die Palette der flüssigkristallinen Substanzen, die sich unter verschiedenen anwendungstechnischen Gesichtspunkten zu Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung zu erniedrigen. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zur Herstellung von Verbindungen der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt,

oder daß man zur Herstellung von Verbindungen der Formel I, worin X F oder Cl bedeutet, in einem entsprechenden Diazoniumsalz die Diazoniumgruppe durch F oder Cl ersetzt,

oder daß man zur Herstellung von Estern der Formel I (worin $Z^2$ und/oder $Z^3$ —CO—O— bedeuten und/oder R und/oder R′ eine Carboxylgruppe enthalten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen der Formel I ein entsprechendes Carbonsäureamid dehydratisiert oder ein entsprechendes Carbonsäurehalogenid mit sulfamid umsetzt,

oder daß man zur Herstellung von Ethern der Formel I (worin R und/oder R′ eine Alkoxygruppe bedeutet und/oder $Z^2$ eine $—CH_2O—$Gruppe ist) eine entsprechende Hydroxyverbindung verethert,

und/oder daß man gegebenenfalls eine Chlorverbindung der Formel I mit einem Cyanid umsetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer einen polarisierbaren stickstoffhaltigen Heterocyclus aufweisenden flüssigkristallinen Verbindung mit dem polarisierenden Strukturelement

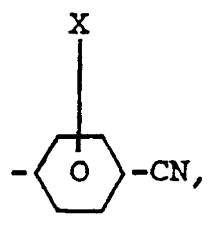

worin X F, Cl, oder $CF_3$ bedeutet, insbesondere einer Verbindung der Formel I, sowie Flüssigkristall-Anzeigeelemente, die derartige Phasen enthalten. Derartige Phasen zeigen besonders vorteilhafte Werte für die optische und dielektrische Anisotropie.

Die Verbindungen der Formel I umfassen dementsprechend Verbindungen mit drei Ringen der Teilformeln Ib

$$R—A^1—A^2—Z^2—Phe—X,CN \qquad Ib$$

sowie Verbindungen mit vier Ringen der Teilformel Ie

$$R'—A^3—Z^3—A^1—A^2—Z^2—Phe—X,CN \qquad Ie$$

In den Verbindungen der vor- und nachstehenden Formeln bedeuten R und R′ vorzugsweise Alkyl, ferner Alkoxy.

Bevorzugt enthält die Verbindung der Formel I nicht mehr als einen der Reste Pyd, Pym oder Pyr.

$Z^2$ ist bevorzugt eine —CO—O—, oder —$CH_2O$— Gruppe, insbesondere bevorzugt ist $Z^2$ —CO—O—.

X bedeutet vorzugsweise in 3-Stellung befindliches F oder Cl, insbesondere F, in zweiter Linie bevorzugt in 2-Stellung befindliches F.

Falls R und/oder R' Alkylreste und/oder Alkoxyreste bedeuten, so können sie geradkettig oder verzweigt sein. Vorzugsweise sind sie geradkettig, haben 2, 3 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 3-Oxapropyl (=Methoxymethyl), 2- (=Ethoxymethyl) oder 3-Oxabutyl (=2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Verbindungen der Formeln I mit verzweigten Flügelgruppen R oder R' können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Rest R und R' sind Isopropyl, 2-Butyl (=1-Methylpropyl), Isobutyl (=2-Methylpropyl), 2-Methylbutyl, Isopentyl (=3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Formel I umfaßt sowohl die Racemate dieser Verbindungen als auch die optischen Antipoden sowie deren Gemische.

Unter den Verbindungen der Formeln I sowie Ib und Ie sind diejenigen bevorzugt, in denen mindestens einer der darin enthaltenen Reste eine der angegebenen bevorzugten Bedeutungen hat.

Besonders bevorzugte kleinere Gruppen von Verbindungen sind diejenigen der Formel I2, I3, I4, I5, I12, I14, I15, I17 und I18, worin Az einer der Ringe Pyd, Pym oder Pyr bedeutet:

R—Phe—Pym—COO—Phe—X,CN I2

R—Phe—Pyr—COO—Phe—X,CN I3

R—Az—Phe—COO—Phe—X,CN I4

R—Az—Cy—COO—Phe—X,CN I5

R'—Pym—COO—Phe—Phe—COO—Phe—X,CN I12

R—Phe—Pym—$CH_2O$—Phe—X,CN I14

R—Phe—Pyr—$CH_2O$—Phe—X,CN I15

R—Az—Cy—$CH_2O$—Phe—X,CN I17

R—Az—Phe—$CH_2O$—Phe—X,CN I18

In den Verbindungen der Formel I sind diejenigen Stereoisomeren bevorzugt, in denen die Ringe Cy trans-1,4-disubstituiert. Diejenigen der vorstehend genannten Formeln, die eine oder mehrere Gruppen Pyd, Pym und/oder Pyr enthalten, umschließen jeweils die beiden 2,5-(Pym, Pyr) Stellungsisomeren.

In den Verbindungen der Formel I, in denen $A^1$ für einen in 2-Stellung durch R substituierten Ring Pyd, Pym oder Pyr steht, bedeutet R bevorzugt Alkyl.

Besonders bevorzugt sind Verbindungen der Formel I worin R und R' jeweils geradkettige oder höchstens einfach verzweigte Alkylgruppen oder Alkoxygruppen mit 1—12, insbesondere 2—10 C-Atomen bedeuten.

Besonders bevorzugt sind die folgenden kleineren Gruppen von Verbindungen, in denen Az Pyd Pyridazin-3,6-diyl oder Pym Pyrimidin-2,5-diyl oder Pyr Pyridin-2,5-diyl, Phe 1,4-Phenylen, Cy 1,4-Cyclohexylen und Phe—X,CN in 2- oder 3-Stellung substituiertes 4-Cyanophenyl bedeutet.

Alkyl bedeutet vorzugsweise geradkettiges Methyl, Ethyl, Propyl, Butyl, Pentyl, Hexyl, Heptyl, Octyl, Nonyl oder Decyl; Oxaalkyl bedeutet vorzugsweise geradkettiges 2-Oxypropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Alkanoyloxy bedeutet vorzugsweise geradkettiges Acetoxy, Propanoyloxy, Butanoyloxy, Pentanoyloxy, Hexanoyloxy, Heptanoyloxy, Octanoyloxy, Nonanoyloxy, Decanoyloxy, Alkoxycarbonyl

bedeutet vorzugsweise geradkettiges Methoxycarbonyl, Ethoxycarbonyl, Propoxycarbonyl, Butoxycarbonyl, Pentoxycarbonyl, Hexoxycarbonyl, Heptoxycarbonyl, Octoxycarbonyl, Nonoxycarbonyl, Decoxycarbonyl.

III.
Alkyl—Phe—Az—COO—Phe—2F,CN
Alkyl—Phe—Az—COO—Phe—3F,CN
Alkyl—Phe—Az—COO—Phe—2Cl,CN
Alkyl—Phe—Az—COO—Phe—3Cl,CN
Alkyl—Phe—Az—COO—Phe—$2CF_3$,CN
Alkyl—Phe—Az—COO—Phe—$3CF_3$,CN
mit Az = Pym oder Pyr

V.
Alkyl—Az—Cy—COO—Phe—2F,CN
Alkyl—Az—Cy—COO—Phe—3F,CN
Alkyl—Az—Cy—COO—Phe—2Cl,CN
Alkyl—Az—Cy—COO—Phe—3Cl,CN
Alkyl—Az—Cy—COO—Phe—$2CF_3$,CN
Alkyl—Az—Cy—COO—Phe—$3CF_3$,CN
mit Az = Pyd, Pym oder Pyr

VI.
Alkyl—Az—Phe—COO—Phe—2F,CN
Alkyl—Az—Phe—COO—Phe—3F,CN
Alkyl—Az—Phe—COO—Phe—2Cl,CN
Alkyl—Az—Phe—COO—Phe—3Cl,CN
Alkyl—Az—Phe—COO—Phe—$2CF_3$,CN
Alkyl—Az—Phe—COO—Phe—$3CF_3$,CN
miz Az = Pyd, Pym oder Pyr

XVI.
Alkyl—Az—COO—Phe—Phe—COO—Phe—2F,CN
Alkyl—Az—COO—Phe—Phe—COO—Phe—3F,CN
Alkyl—Az—COO—Phe—Phe—COO—Phe—2Cl,CN
Alkyl—Az—COO—Phe—Phe—COO—Phe—3Cl,CN
Alkyl—Az—COO—Phe—Phe—COO—Phe—$2CF_3$,CN
Alkyl—Az—COO—Phe—Phe—COO—Phe—$3CF_3$,CN
miz Az = Pym

XVIII.
Alkyl—Phe—Az—$CH_2O$—Phe—2F,CN
Alkyl—Phe—Az—$CH_2O$—Phe—3F,CN
Alkyl—Phe—Az—$CH_2O$—Phe—2Cl,CN
Alkyl—Phe—Az—$CH_2O$—Phe—3Cl,CN
Alkyl—Phe—Az—$CH_2O$—Phe—$2CF_3$,CN
Alkyl—Phe—Az—$CH_2O$—Phe—$3CF_3$,CN
mit Az = Pym oder Pyr

XIX.
Alkylcarbonyloxy—Phe—Az—$CH_2O$—Phe—2F,CN
Alkylcarbonyloxy—Phe—Az—$CH_2O$—Phe—3F,CN
Alkylcarbonyloxy—Phe—Az—$CH_2O$—Phe—2Cl,CN
Alkylcarbonyloxy—Phe—Az—$CH_2O$—Phe—3Cl,CN
Alkylcarbonyloxy—Phe—Az—$CH_2O$—Phe—$2CF_3$,CN
Alkylcarbonyloxy—Phe—Az—$CH_2O$—Phe—$3CF_3$,CN
mit Az = Pym oder Pyr

XXI.
Alkyl—Az—Cy—$CH_2O$—Phe—2F,CN
Alkyl—Az—Cy—$CH_2O$—Phe—3F,CN
Alkyl—Az—Cy—$CH_2O$—Phe—2Cl,CN
Alkyl—Az—Cy—$CH_2O$—Phe—3Cl,CN
Alkyl—Az—Cy—$CH_2O$—Phe—2CF,CN
Alkyl—Az—Cy—$CH_2O$—Phe—$3CF_3$,CN
mit Az = Pyd, Pym oder Pyr

XXII.
Alkyl—Az—Phe—$CH_2O$—Phe—2F,CN
Alkyl—Az—Phe—$CH_2O$—Phe—3F,CN
Alkyl—Az—Phe—$CH_2O$—Phe—2Cl,CN
Alkyl—Az—Phe—$CH_2O$—Phe—3Cl,CN
Alkyl—Az—Phe—$CH_2O$—Phe—$2CF_3$,CN

Alkyl—Az—Phe—$CH_2O$—Phe—$3CF_3$,CN
mit Az = Pyd, Pym oder Pyr

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem eine Verbindung, die sonst der Formel I entspricht, aber an Stelle von H-Atomen eine oder mehrere reduzierbare Gruppen und/oder C—C-Bindungen enthält, reduziert.

Als reduzierbare Gruppen kommen vorzugsweise Carbonylgruppen in Betracht, insbesondere Ketrogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer —$CH_2CH_2$-Gruppe eine —CH=CH-Gruppe und/oder an Stelle einer —$CH_2$-Gruppe eine —CO-Gruppe und/oder an Stelle eines H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder —$CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Akylester mit 1—4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Keton wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder

Schwefelsäure, umgesetzt. Eine bevorzugt Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Collidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa −25° und +20°.

Zur Herstellung von Nitrilen der Formel I können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin R und/oder R′ eine Alkoxygruppe bedeutet und/oder $Z^2$ eine —$CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metall derivat, z.B. durch Behandeln mit NaH, $NaNH_2$, NaOH, KOH, $Na_2CO_3$ oder $K_2CO_3$ in das entsprechende Akalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH und KOH bei Temperaturen zwischen etwa 20° und 100°.

Zur Herstellung von Nitrilen der Formel I können auch entsprechende Chlor- und Bromverbindungen der Formel I mit einem Cyanid umgesetzt werden, zweckmäßig mit einem Metallcyanid wie NaCN, KCN oder $Cu_2(CN)_2$, z.B. in Gegenwart von Pyridin in einem inerten Lösungsmittel wie DMF oder N-Methylpyrrolidon bei Temperaturen zwischen 20° und 200°.

Verbindungen der Formel I, worin X F oder Cl bedeutet, können auch aus den entsprechenden Diazoniumsalzen durch Austausch der Diazoniumgruppe gegen ein Fluor- oder Chloratom z.B. nach den Methoden von Schiemann oder Sandmeyer, erhalten werden.

Die Diazoniumsalze sind z.B. herstellbar durch Nitrierung von Verbindungen, die der Formel I entsprechen, aber an Stelle des Restes X ein Wasserstoffatom enthalten, Reduktion zu den entsprechenden Aminen und Diazotierung beispielsweise mit $NaNO_2$ oder $KNO_2$ in wässeriger Lösung bei Temperaturen zwischen etwa −10 und +10°.

Zum Austausch der Diazoniumgruppe gegen Fluor kann man in wasserfreier Flußsäure diazotieren und anschließend erwärmen, oder man setzt mit Tetrafluorborsäure zu den Diazoniumtetrafluorboraten um, die anschließend thermisch zersetzt werden.

Ein Austauschgegen Cl gelingt zweckmäßig durch Reaktion der wässerigen Diazoniumsalzlösung mit $Cu_2Cl_2$ nach der Methode von Sandmeyer.

Verbindungen der Formel I, worin X $CF_3$ bedeutet, können aus Carbonsäuren oder deren Derivaten wie z.B. Säurehalogeniden oder Anhydriden mit Schwefeltetrafluorid bei erhöhter Temperatur erhalten werden.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäure-phenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4′-Biscyclohexylbiphenyle, Phenyl- oder Cyclohexylpyrimidine, Phenyl- oder Cyclohexyldioxane, Phenyl- oder Cyclohexyldithiane, 1,2-Bis-phenylethane, 1,2-Biscyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$R^1—L—G—E—R^2$ II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituierten Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO— |

oder eine C—C-Einfachbindung, Y Halogen, vorzugsweise Chlor, oder —CN, und $R^1$ und $R^2$ Alkyl, Alkoxy, Alkanoyloxy oder Alkoxycarbonyloxy mit bis zu 18, vorzugsweise bis zu 8 Kohlenstoffatomen, oder einer dieser Reste auch CN, $NO_2$, $CF_3$, F, Cl oder Br bedeuten.

Bei den meisten dieser Verbindungen sind $R^1$ und $R^2$ voneinander verschieden, wobei einer dieser Reste meist eine Alkyl- oder Alkoxygruppe ist. Aber auch andere Varianten der vorgesehen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen flüssigkristallinen Phasen enthalten etwa 0,1 bis 99, vorzugsweise 10 bis 95%, einer oder mehrerer Verbindungen der Formel I. Weiterhin bevorzugt sind flüssigkristalline Phasen, die 0,1—50, insbesondere 0,5—30% einer oder mehrerer Verbindungen der Formel I enthalten. Auch isotrope Verbindungen der Formel I können in den erfindungsgemäßen Phasen verwendet werden.

Die Herstellung der erfindungsgemäßen flüssigkristallinen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band 24, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE—OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern. Vor- und nachstehend bedeuten Prozentangaben Gewichtsprozent. Alle Temperaturen sind in Grad Celsius angegeben.

Beispiel 1

29,8 g 4-(5-Heptylpyrimidin-2-yl)-benzoesäure (erhältlich aus 4-Carbo-methoxybenzamidinhydrochlorid und Heptylmalondialdehydtetramethylacetal mit anschließender Verseifung des Methylesters) werden durch Erhitzen mit 15 g Thionylchlorid in das Säurechlorid überführt. Man dampft eine, löst den Rückstand in 350 ml Toluol, versetzt mit 25 ml Pyridin und 13,7 g 3-Fluor-4-cyanphenol (JP—OS 57—050953) und kocht 2 Stunden. Das Gemisch wird mit Wasser versetzt. Man trennt ab, wäscht die organische Phase mit Wasser, trocknet über Natriumsulfat, dampft eine und erhält 4-(5-Heptylpyrimidin-2-yl)-benzoesäure-(3-fluor-4-cyan-phenyl)-ester.

Die Verbindung zeigt folgende Phasenübergänge:

kristallin $\overset{82°}{\longleftrightarrow}$ smektisch A $\overset{149°}{\longleftrightarrow}$ nematisch $\overset{198°}{\longleftrightarrow}$ isotrop

Analog werden die nachfolgend und in den Gruppen VI und XXII angegebenen Verbindungen erhalten:

4-(5-Methylpyrimidin-2-yl)-benzoesäure-(2-fluor-4-cyanphenyl)-ester
4-(5-Ethylpyrimidin-2-yl)-benzoesäure-(2-fluor-4-cyanphenyl)-ester

4-(5-Propylpyrimidin-2-yl)-benzoesäure-(2-fluor-4-cyanphenyl)-ester
4-(5-Butylpyrimidin-2-yl)-benzoesäure-(2-fluor-4-cyanphenyl)-ester
4-(5-Pentylpyrimidin-2-yl)-benzoesäure-(2-fluor-4-cyanphenyl)-ester
4-(5-Hexylpyrimidin-2-yl)-benzoesäure-(2-fluor-4-cyanphenyl)-ester
4-(5-Heptylpyrimidin-2-yl)-benzoesäure-(2-fluor-4-cyanphenyl)-ester
4-(5-Octylpyrimidin-2-yl)-benzoesäure-(2-fluor-4-cyanphenyl)-ester
4-(5-Nonylpyrimidin-2-yl)-benzoesäure-(2-fluor-4-cyanphenyl)-ester
4-(5-Decylpyrimidin-2-yl)-benzoesäure-(2-fluor-4-cyanphenyl)-ester
4-(5-Undecylpyrimidin-2-yl)-benzoesäure-(2-fluor-4-cyanphenyl)-ester
4-(5-Dodecylpyrimidin-2-yl)-benzoesäure-(2-fluor-4-cyanphenyl)-ester
4-(5-Methylpyrimidin-2-yl)-benzoesäure-(3-fluor-4-cyanphenyl)-ester
4-(5-Ethylpyrimidin-2-yl)-benzoesäure-(3-fluor-4-cyanphenyl)-ester
4-(5-Propylpyrimidin-2-yl)-benzoesäure-(3-fluor-4-cyanphenyl)-ester
4-(5-Butylpyrimidin-2-yl)-benzoesäure-(3-fluor-4-cyanphenyl)-ester
4-(5-Pentylpyrimidin-2-yl)-benzoesäure-(3-fluor-4-cyanphenyl)-ester
4-(5-Hexylpyrimidin-2-yl)-benzoesäure-(3-fluor-4-cyanphenyl)-ester
4-(5-Heptylpyrimidin-2-yl)-benzoesäure-(2-fluor-4-cyanphenyl)-ester
4-(5-Octylpyrimidin-2-yl)-benzoesäure-(3-fluor-4-cyanphenyl)-ester
4-(5-Nonylpyrimidin-2-yl)-benzoesäure-(3-fluor-4-cyanphenyl)-ester
4-(5-Decylpyrimidin-2-yl)-benzoesäure-(3-fluor-4-cyanphenyl)-ester
4-(5-Undecylpyrimidin-2-yl)-benzoesäure-(3-fluor-4-cyanphenyl)-ester
4-(5-Dodecylpyrimidin-2-yl)-benzoesäure-(3-fluor-4-cyanphenyl)-ester
4-(5-Methylpyridin-2-yl)-benzoesäure-(3-fluor-4-cyanphenyl)-ester
4-(5-Ethylpyridin-2-yl)-benzoesäure-(3-fluor-4-cyanphenyl)-ester
4-(5-Propylpyridin-2-yl)-benzoesäure-(3-fluor-4-cyanphenyl)-ester
4-(5-Butylpyridin-2-yl)-benzoesäure-(3-fluor-4-cyanphenyl)-ester
4-(5-Pentylpyridin-2-yl)-benzoesäure-(3-fluor-4-cyanphenyl)-ester
4-(5-Hexylpyridin-2-yl)-benzoesäure-(3-fluor-4-cyanphenyl)-ester
4-(5-Heptylpyridin-2-yl)-benzoesäure-(3-fluor-4-cyanphenyl)-ester
4-(5-Octylpyridin-2-yl)-benzoesäure-(3-fluor-4-cyanphenyl)-ester
4-(5-Nonylpyridin-2-yl)-benzoesäure-(3-fluor-4-cyanphenyl)-ester
4-(5-Decylpyridin-2-yl)-benzoesäure-(3-fluor-4-cyanphenyl)-ester
4-(5-Undecylpyridin-2-yl)-benzoesäure-(3-fluor-4-cyanphenyl)-ester
4-(5-Dodecylpyridin-2-yl)-benzoesäure-(3-fluor-4-cyanphenyl)-ester
4-(6-Methylpyridazin-3-yl)-benzoesäure-(3-fluor-4-cyanphenyl)-ester
4-(6-Ethylpyridazin-3-yl)-benzoesäure-(3-fluor-4-cyanphenyl)-ester
4-(6-Propylpyridazin-3-yl)-benzoesäure-(3-fluor-4-cyanphenyl)-ester
4-(6-Butylpyridazin-3-yl)-benzoesäure-(3-fluor-4-cyanphenyl)-ester
4-(6-Pentylpyridazin-3-yl)-benzoesäure-(3-fluor-4-cyanphenyl)-ester
4-(6-Hexylpyridazin-3-yl)-benzoesäure-(3-fluor-4-cyanphenyl)-ester
4-(6-Heptylpyridazin-3-yl)-benzoesäure-(3-fluor-4-cyanphenyl)-ester
4-(6-Octylpyridazin-3-yl)-benzoesäure-(3-fluor-4-cyanphenyl)-ester
4-(6-Nonylpyridazin-3-yl)-benzoesäure-(3-fluor-4-cyanphenyl)-ester
4-(6-Decylpyridazin-3-yl)-benzoesäure-(3-fluor-4-cyanphenyl)-ester
4-(6-Undecylpyridazin-3-yl)-benzoesäure-(3-fluor-4-cyanphenyl)-ester
4-(6-Dodecylpyridazin-3-yl)-benzoesäure-(3-fluor-4-cyanphenyl)-ester
(4-(5-Methylpyrimidin-2-yl)-phenylmethyl)-(2-fluor-4-cyanphenyl)-ether
(4-(5-Ethylpyrimidin-2-yl)-phenylmethyl)-(2-fluor-4-cyanphenyl)-ether
(4-(5-Propylpyrimidin-2-yl)-phenylmethyl)-(2-fluor-4-cyanphenyl)-ether
(4-(5-Butylpyrimidin-2-yl)-phenylmethyl)-(2-fluor-4-cyanphenyl)-ether
(4-(5-Pentylpyrimidin-2-yl)-phenylmethyl)-(2-fluor-4-cyanphenyl)-ether
(4-(5-Hexylpyrimidin-2-yl)-phenylmethyl)-(2-fluor-4-cyanphenyl)-ether
(4-(5-Heptylpyrimidin-2-yl)-phenylmethyl)-(2-fluor-4-cyanphenyl)-ether
(4-(5-Octylpyrimidin-2-yl)-phenylmethyl)-(2-fluor-4-cyanphenyl)-ether
(4-(5-Nonylpyrimidin-2-yl)-phenylmethyl)-(2-fluor-4-cyanphenyl)-ether
(4-(5-Decylpyrimidin-2-yl)-phenylmethyl)-(2-fluor-4-cyanphenyl)-ether
(4-(5-Undecylpyrimidin-2-yl)-phenylmethyl)-(2-fluor-4-cyanphenyl)-ether
(4-(5-Dodecylpyrimidin-2-yl)-phenylmethyl)-(2-fluor-4-cyanphenyl)-ether
(4-(5-Methylpyrimidin-2-yl)-phenylmethyl)-(3-trifluormethyl-4-cyanphenyl)-ether
(4-(5-Ethylpyrimidin-2-yl)-phenylmethyl)-(3-trifluormethyl-4-cyanphenyl)-ether
(4-(5-Propylpyrimidin-2-yl)-phenylmethyl)-(3-trifluormethyl-4-cyanphenyl)-ether
(4-(5-Butylpyrimidin-2-yl)-phenylmethyl)-(3-trifluormethyl-4-cyanphenyl)-ether
(4-(5-Pentylpyrimidin-2-yl)-phenylmethyl)-(3-trifluormethyl-4-cyanphenyl)-ether
(4-(5-Hexylpyrimidin-2-yl)-phenylmethyl)-(3-trifluormethyl-4-cyanphenyl)-ether
(4-(5-Heptylpyrimidin-2-yl)-phenylmethyl)-(3-trifluormethyl-4-cyanphenyl)-ether

(4-(5-Octylpyrimidin-2-yl)-phenylmethyl)-(3-trifluormethyl-4-cyanphenyl)-ether
(4-(5-Nonylpyrimidin-2-yl)-phenylmethyl)-(3-trifluormethyl-4-cyanphenyl)-ether
(4-(5-Decylpyrimidin-2-yl)-phenylmethyl)-(3-trifluormethyl-4-cyanphenyl)-ether
(4-(5-Undecylpyrimidin-2-yl)-phenylmethyl)-(3-trifluormethyl-4-cyanphenyl)-ether
(4-(5-Dodecylpyrimidin-2-yl)-phenylmethyl)-(3-trifluormethyl-4-cyanphenyl)-ether
(4-(5-Methylpyrimidin-2-yl)-phenylmethyl)-(3-fluor-4-cyanphenyl)-ether
(4-(5-Ethylpyrimidin-2-yl)-phenylmethyl)-(3-fluor-4-cyanphenyl)-ether
(4-(5-Undecylpyrimidin-2-yl)-phenylmethyl)-(3-fluor-4-cyanphenyl)-ether
(4-(5-Methylpyridin-2-yl)-phenylmethyl)-(3-fluor-4-cyanphenyl)-ether
(4-(5-Ethylpyridin-2-yl)-phenylmethyl)-(3-fluor-4-cyanphenyl)-ether
(4-(5-Propylpyridin-2-yl)-phenylmethyl)-(3-fluor-4-cyanphenyl)-ether
(4-(5-Butylpyridin-2-yl)-phenylmethyl)-(3-fluor-4-cyanphenyl)-ether
(4-(5-Pentylpyridin-2-yl)-phenylmethyl)-(3-fluor-4-cyanphenyl)-ether
(4-(5-Hexylpyridin-2-yl)-phenylmethyl)-(3-fluor-4-cyanphenyl)-ether
(4-(5-Heptylpyridin-2-yl)-phenylmethyl)-(3-fluor-4-cyanphenyl)-ether
(4-(5-Octylpyridin-2-yl)-phenylmethyl)-(3-fluor-4-cyanphenyl)-ether
(4-(5-Nonylpyridin-2-yl)-phenylmethyl)-(3-fluor-4-cyanphenyl)-ether
(4-(5-Decylpyridin-2-yl)-phenylmethyl)-(3-fluor-4-cyanphenyl)-ether
(4-(5-Undecylpyridin-2-yl)-phenylmethyl)-(3-fluor-4-cyanphenyl)-ether
(4-(5-Dodecylpyridin-2-yl)-phenylmethyl)-(3-fluor-4-cyanphenyl)-ether

Beispiel 2

30,8 g trans-4-(5-Pentylpyrimidin-2-yl)-cyclohexancarbonsäure (erhältlich aus Pentylmalondialdehydtetramethylacetal und trans-4-Carbomethoxycyclohexancarbamidinhydrochlorid mit anschließender alkalischer Verseifung der Estergruppe) und 13,7 g 3-Fluor-4-cyanphenol werden in 500 ml Dichlormethan vorglegt und portionsweise mit 21 g Dicyclohexylcarbodiimid versetzt. Man rührt 2 Stunden bei 20°, saugt von ausgefallenem Harnstoff ab und engt auf ein kleines Volumen ein. Nach adsorptiver Filtration des Rückstandes über Kieselgel mit Ethylacetat aus Elutionsmittel wird trans-4-(5-Pentylpyrimidin-2-yl)-cyclohexancarbonsäure-(3-fluor-4-cyanphenyl)-ester erhalten.

Analog werden die nachfolgend und in den Gruppen V, genannten Verbindungen erhalten:

trans-4-(5-Methylpyrimidin-2-yl)-cyclohexancarbonsäure-(2-fluor-4-cyanphenyl)-ester
trans-4-(5-Ethylpyrimidin-2-yl)-cyclohexancarbonsäure-(2-fluor-4-cyanphenyl)-ester
trans-4-(5-Propylpyrimidin-2-yl)-cyclohexancarbonsäure-(2-fluor-4-cyanphenyl)-ester
trans-4-(5-Butylpyrimidin-2-yl)-cyclohexancarbonsäure-(2-fluor-4-cyanphenyl)-ester
trans-4-(5-Pentylpyrimidin-2-yl)-cyclohexancarbonsäure-(2-fluor-4-cyanphenyl)-ester
trans-4-(5-Hexylpyrimidin-2-yl)-cyclohexancarbonsäure-(2-fluor-4-cyanphenyl)-ester
trans-4-(5-Heptylpyrimidin-2-yl)-cyclohexancarbonsäure-(2-fluor-4-cyanphenyl)-ester
trans-4-(5-Octylpyrimidin-2-yl)-cyclohexancarbonsäure-(2-fluor-4-cyanphenyl)-ester
trans-4-(5-Nonylpyrimidin-2-yl)-cyclohexancarbonsäure-(2-fluor-4-cyanphenyl)-ester
trans-4-(5-Decylpyrimidin-2-yl)-cyclohexancarbonsäure-(2-fluor-4-cyanphenyl)-ester
trans-4-(5-Undecylpyrimidin-2-yl)-cyclohexancarbonsäure-(2-fluor-4-cyanphenyl)-ester
trans-4-(5-Dodecylpyrimidin-2-yl)-cyclohexancarbonsäure-(2-fluor-4-cyanphenyl)-ester
trans-4-(5-Methylpyrimidin-2-yl)-cyclohexancarbonsäure-(3-fluor-4-cyanphenyl)-ester
trans-4-(5-Ethylpyrimidin-2-yl)-cyclohexancarbonsäure-(3-fluor-4-cyanphenyl)-ester
trans-4-(5-Propylpyrimidin-2-yl)-cyclohexancarbonsäure-(3-fluor-4-cyanphenyl)-ester
trans-4-(5-Butylpyrimidin-2-yl)-cyclohexancarbonsäure-(3-fluor-4-cyanphenyl)-ester
trans-4-(5-Pentylpyrimidin-2-yl)-cyclohexancarbonsäure-(3-fluor-4-cyanphenyl)-ester
trans-4-(5-Hexylpyrimidin-2-yl)-cyclohexancarbonsäure-(3-fluor-4-cyanphenyl)-ester
trans-4-(5-Heptylpyrimidin-2-yl)-cyclohexancarbonsäure-(3-fluor-4-cyanphenyl)-ester
trans-4-(5-Octylpyrimidin-2-yl)-cyclohexancarbonsäure-(3-fluor-4-cyanphenyl)-ester
trans-4-(5-Nonylpyrimidin-2-yl)-cyclohexancarbonsäure-(3-fluor-4-cyanphenyl)-ester
trans-4-(5-Decylpyrimidin-2-yl)-cyclohexancarbonsäure-(3-fluor-4-cyanphenyl)-ester
trans-4-(5-Undecylpyrimidin-2-yl)-cyclohexancarbonsäure-(3-fluor-4-cyanphenyl)-ester
trans-4-(5-Dodecylpyrimidin-2-yl)-cyclohexancarbonsäure-(3-fluor-4-cyanphenyl)-ester
trans-4-(6-Methylpyridazin-3-yl)-cyclohexancarbonsäure-(3-fluor-4-cyanphenyl)-ester
trans-4-(6-Ethylpyridazin-3-yl)-cyclohexancarbonsäure-(3-fluor-4-cyanphenyl)-ester
trans-4-(6-Propylpyridazin-3-yl)-cyclohexancarbonsäure-(3-fluor-4-cyanphenyl)-ester
trans-4-(6-Butylpyridazin-3-yl)-cyclohexancarbonsäure-(3-fluor-4-cyanphenyl)-ester
trans-4-(6-Pentylpyridazin-3-yl)-cyclohexancarbonsäure-(3-fluor-4-cyanphenyl)-ester
trans-4-(6-Hexylpyridazin-3-yl)-cyclohexancarbonsäure-(3-fluor-4-cyanphenyl)-ester
trans-4-(6-Heptylpyridazin-3-yl)-cyclohexancarbonsäure-(3-fluor-4-cyanphenyl)-ester
trans-4-(6-Octylpyridazin-3-yl)-cyclohexancarbonsäure-(3-fluor-4-cyanphenyl)-ester
trans-4-(6-Nonylpyridazin-3-yl)-cyclohexancarbonsäure-(3-fluor-4-cyanphenyl)-ester
trans-4-(6-Decylpyridazin-3-yl)-cyclohexancarbonsäure-(3-fluor-4-cyanphenyl)-ester

trans-4-(6-Undecylpyridazin-3-yl)-cyclohexancarbonsäure-(3-fluor-4-cyanphenyl)-ester
trans-4-(6-Dodecylpyridazin-3-yl)-cyclohexancarbonsäure-(3-fluor-4-cyanphenyl)-ester
trans-4-(5-Methylpyridin-2-yl)-cyclohexancarbonsäure-(3-fluor-4-cyanphenyl)-ester
trans-4-(5-Ethylpyridin-2-yl)-cyclohexancarbonsäure-(3-fluor-4-cyanphenyl)-ester
trans-4-(5-Propylpyridin-2-yl)-cyclohexancarbonsäure-(3-fluor-4-cyanphenyl)-ester
trans-4-(5-Butylpyridin-2-yl)-cyclohexancarbonsäure-(3-fluor-4-cyanphenyl)-ester
trans-4-(5-Pentylpyridin-2-yl)-cyclohexancarbonsäure-(3-fluor-4-cyanphenyl)-ester
trans-4-(5-Hexylpyridin-2-yl)-cyclohexancarbonsäure-(3-fluor-4-cyanphenyl)-ester
trans-4-(5-Heptylpyridin-2-yl)-cyclohexancarbonsäure-(3-fluor-4-cyanphenyl)-ester
trans-4-(5-Octylpyridin-2-yl)-cyclohexancarbonsäure-(3-fluor-4-cyanphenyl)-ester
trans-4-(5-Nonylpyridin-2-yl)-cyclohexancarbonsäure-(3-fluor-4-cyanphenyl)-ester
trans-4-(5-Decylpyridin-2-yl)-cyclohexancarbonsäure-(3-fluor-4-cyanphenyl)-ester
trans-4-(5-Undecylpyridin-2-yl)-cyclohexancarbonsäure-(3-fluor-4-cyanphenyl)-ester
trans-4-(5-Dodecylpyridin-2-yl)-cyclohexancarbonsäure-(3-fluor-4-cyanphenyl)-ester

Beispiel 3

3,8 g 5-Heptylpyrimidin-2-carbonsäure-(4-bromphenyl)-ester (erhältlich aus 4-Bromphenol und 5-Heptylpyrimidin-2-carbonsäure analog Beispiel 2) und 3,2 g 4-Brombenzoesäure-(3-fluor-4-cyanphenyl)-ester (erhältlich aus 4-Brombenzoesäure und 3-Fluor-4-cyanphenol analog Beispiel 1) werden nach Zusatz von 1 g Kupferbronze 15 Stunden auf 140° erhitzt. Das Reaktionsgemisch wird hernach in Ether aufgenommen, von Unlöslichem filtriert und zum Rückstand eingeengt. Nach chromatographischer Aufreinigung erhält man 4'-(5-Heptylpyrimidin-2-ylcarbonyloxy)-biphenyl-4-carbonsäure-(3-fluor-4-cyanphenyl)-ester. Analog lassen sich die in Gruppe XVI angegebenen Verbindungen erhalten.

Beispiel 4

2,8 g 4-(5-Heptylpyrimidin-2-yl)-benzylalkohol (erhältlich aus der in Beispiel 1 beschriebenen 4-(5-Heptylpyrimidin-2-yl)-benzoesäure mit Lithiumaluminiumhydrid in siedendem Dioxan) werden in 30 ml Benzol gelöst und bei 20° mit 1,0 g Phosphortribromid behandelt. Nach Abdekantieren von entstandener phosphoriger Säure engt man die Reaktionslösung zum Rückstand ein und nimmt in 40 ml Dimethylformamid auf. Es werden 1,4 g 3-Fluor-4-cyanphenol und 1 g gepulvertes Kaliumcarbonat zugegeben und anschließend 5 Stdn. bei 35° gerührt. Zur Aufarbeitung wird auf 300 ml Wasser gegossen und mit Ether extrahiert. Nach Waschen der Extrakte mit Wasser und Trocknen über Natriumsulfat engt man ein und kristallisiert den Rückstand um.

Man erhält so (4-(5-Heptylpyrimidin-2-yl)-benzyl-(3-fluor-4-cyanphenyl)-ether.

Analog werden die nachfolgend und in den Gruppen XX bis XXII beschriebenen Verbindungen erhalten:

(trans-4-(5-Methylpyrimidin-2-yl)-cyclohexylmethyl)-(2-fluor-4-cyanphenyl)-ether
(trans-4-(5-Ethylpyrimidin-2-yl)-cyclohexylmethyl)-(2-fluor-4-cyanphenyl)-ether
(trans-4-(5-Propylpyrimidin-2-yl)-cyclohexylmethyl)-(2-fluor-4-cyanphenyl)-ether
(trans-4-(5-Butylpyrimidin-2-yl)-cyclohexylmethyl)-(2-fluor-4-cyanphenyl)-ether
(trans-4-(5-Pentylpyrimidin-2-yl)-cyclohexylmethyl)-(2-fluor-4-cyanphenyl)-ether
(trans-4-(5-Hexylpyrimidin-2-yl)-cyclohexylmethyl)-(2-fluor-4-cyanphenyl)-ether
(trans-4-(5-Heptylpyrimidin-2-yl)-cyclohexylmethyl)-(2-fluor-4-cyanphenyl)-ether
(trans-4-(5-Octylpyrimidin-2-yl)-cyclohexylmethyl)-(2-fluor-4-cyanphenyl)-ether
(trans-4-(5-Nonylpyrimidin-2-yl)-cyclohexylmethyl)-(2-fluor-4-cyanphenyl)-ether
(trans-4-(5-Decylpyrimidin-2-yl)-cyclohexylmethyl)-(2-fluor-4-cyanphenyl)-ether
(trans-4-(5-Undecylpyrimidin-2-yl)-cyclohexylmethyl)-(2-fluor-4-cyanphenyl)-ether
(trans-4-(5-Dodecylpyrimidin-2-yl)-cyclohexylmethyl)-(2-fluor-4-cyanphenyl)-ether
(trans-4-(5-Methylpyrimidin-2-yl)-cyclohexylmethyl)-(3-fluor-4-cyanphenyl)-ether
(trans-4-(5-Ethylpyrimidin-2-yl)-cyclohexylmethyl)-(3-fluor-4-cyanphenyl)-ether
(trans-4-(5-Propylpyrimidin-2-yl)-cyclohexylmethyl)-(3-fluor-4-cyanphenyl)-ether
(trans-4-(5-Butylpyrimidin-2-yl)-cyclohexylmethyl)-(3-fluor-4-cyanphenyl)-ether
(trans-4-(5-Hexylpyrimidin-2-yl)-cyclohexylmethyl)-(3-fluor-4-cyanphenyl)-ether
(trans-4-(5-Octylpyrimidin-2-yl)-cyclohexylmethyl)-(3-fluor-4-cyanphenyl)-ether
(trans-4-(5-Undecylpyrimidin-2-yl)-cyclohexylmethyl)-(3-fluor-4-cyanphenyl)-ether
(trans-4-(5-Methylpyridin-2-yl)-cyclohexylmethyl)-(3-fluor-4-cyanphenyl)-ether
(trans-4-(5-Ethylpyridin-2-yl)-cyclohexylmethyl)-(3-fluor-4-cyanphenyl)-ether
(trans-4-(5-Propylpyridin-2-yl)-cyclohexylmethyl)-(3-fluor-4-cyanphenyl)-ether
(trans-4-(5-Butylpyridin-2-yl)-cyclohexylmethyl)-(3-fluor-4-cyanphenyl)-ether
(trans-4-(5-Pentylpyridin-2-yl)-cyclohexylmethyl)-(3-fluor-4-cyanphenyl)-ether
(trans-4-(5-Hexylpyridin-2-yl)-cyclohexylmethyl)-(3-fluor-4-cyanphenyl)-ether
(trans-4-(5-Heptylpyridin-2-yl)-cyclohexylmethyl)-(3-fluor-4-cyanphenyl)-ether
(trans-4-(5-Octylpyridin-2-yl)-cyclohexylmethyl)-(3-fluor-4-cyanphenyl)-ether
(trans-4-(5-Nonylpyridin-2-yl)-cyclohexylmethyl)-(3-fluor-4-cyanphenyl)-ether
(trans-4-(5-Decylpyridin-2-yl)-cyclohexylmethyl)-(3-fluor-4-cyanphenyl)-ether

(trans-4-(5-Undecylpyridin-2-yl)-cyclohexylmethyl)-(3-fluor-4-cyanphenyl)-ether
(trans-4-(5-Dodecylpyridin-2-yl)-cyclohexylmethyl)-(3-fluor-4-cyanphenyl)-ether
Es folgen Beispiele für erfindungsgemäße Mischungen.

Beispiel A

Man stellt ein Gemisch her aus:
25% 4-(5-Heptylpyrimidin-2-yl)-benzoesäure-3-(fluor-4-cyanphenyl)-ester
20% trans-4-(4-Propylcyclohexyl)-benzonitril
20% trans-4-(4-Pentylcyclohexyl)-benzonitril
15% trans-4-(4-Ethoxyphenyl)-propylcyclohexan
10% trans-4-(4-Butoxyphenyl)-propylcyclohexan
10% 4-(trans-4-Pentylcyclohexyl)-4'-(trans-4-propylcyclohexyl)-biphenyl

Beispiel B

Man stellt ein Gemisch her aus:
28% 5-(4-Pentylphenyl)-pyrimidin-2-carbonsäure-(3-fluor-4-cyanphenyl)-ester
26% 5-(4-Pentylcyclohexyl)-pyrimidin-2-carbonsäure-(3-fluor-4-cyanphenyl)-ester
14% trans-4-(4-Pentylcyclohexyl)-cyclohexancarbonsäure-(4-propylcyclohexyl)-ester
12% trans-4-Heptylcyclohexancarbonsäure-(4-cyanphenyl)-ester
10% 4-Butoxybenzoesäure-(4-cyanphenyl]-ester
10% 4-(4-Hexylcyclohexyl)-cyanocyclohexan

Beispiel C

Man stellt ein Gemisch her aus:
24% trans-4-(4-Heptylpyridin-2-yl)-cyclohexancarbonsäure-(2-fluor-4-cyanphenyl)-ester
24% trans-4-(5-Pentylpyridin-2-yl)-cyclohexancarbonsäure-(2-fluor-4-cyanphenyl)-ester
12% 2-(4-Propoxycyclohexyl)-5-pentyl-1,3-dioxan
12% 2-(4-Cyanphenyl)-5-hexylpyrimidin
10% 4-(4-(4-Propoxycyclohexyl)-cyclohexyl)-heptylcyclohexan
10% 4-Butylbenzoesäure-(3-fluor-4-cyanphenyl)-ester
8% 1-(4-Butylcyclohexyl)-2-(4-(4-hexylcyclohexyl)-biphenyl-4-yl)-ethan

Beispiel D

Man stellt ein Gemisch her aus:
26% 4'-(5-Pentylpyrimidin-2-ylcarbonyloxy)-biphenyl-4-carbonsäure-(3-fluor-4-cyanphenyl)-ester
24% 4'-(5-Pentylpyrimidin-2-ylcarbonyloxy)-biphenyl-4-carbonsäure-(3-trifluormethyl-4-cyanphenyl)-ester
15% 4-(4-Propoxycyclohexyl)-pentylcyclohexan
15% 2-(4-Cyanphenyl)-5-(4-butylcyclohexyl)-pyrimidin
12% 4-(Cyano-4'-(4-propylcyclohexyl)-biphenyl
8% 4-(4-(4-Fluor-4-cyanophenyl)-cyclohexyl-butylcyclohexan

Beispiel E

Man stellt ein Gemisch her aus:
34% 5-(2-Fluor-4-heptylphenyl)-pyrimidin-2-carbonsäure-(3-fluor-4-cyanphenyl)-ester
23% 4-Heptyl-4'-(4-ethylcyclohexyl)-biphenyl
17% 2-(4-Butoxyphenyl-5-(4-ethylcyclohexyl)-pyrimidin
13% 4-(4-Pentanoyloxycyclohexyl)-propylcyclohexan
8% 1-Cyan-1-(4-butylcyclohexyl)-4-(4-ethylcyclohexyl)-cyclohexan
5% 1-(4-Propylcyclohexyl)-2-(4-(4-cyanphenyl)-cyclohexyl)-ethan

Beispiel F

Man stellt ein Gemisch her aus:
36% trans-4-(4-Cyanphenyl)-pentylcyclohexan
20% trans-4-(5-Propylpyrimidin-2-yl)-cyclohexancarbonsäure-(3-fluor-4-cyanphenyl)-ester
18% trans-4-(4-Pentylpyrimidin-2-yl)-cyclohexancarbonsäure-(3-fluor-4-cyanphenyl)-ester
17% trans-4-(5-Hexylpyrimidin-2-yl)-cyclohexancarbonsäure-(3-fluor-4-cyanphenyl)-ester
9% trans-4-(4-Cyanbiphenyl-4'-yl)-pentylcyclohexan

Beispiel G

Man stellt ein Gemisch her aus:
32% 4-(5-Hexylpyrimidin-2-yl)-benzoesäure-(3-fluor-4-cyanphenyl)-ester
24% 2-(4-Butoxyphenyl)-5-pentyl-pyrimidin
19% 2-(4-Propylcyclohexyl)-5-heptyl-1,3-dioxan
15% 4-(4-Hexylcyclohexyl)-benzoesäure-(4-cyanphenyl)-ester

11% 1-Cyan-1-(4-butylcyclohexyl)-2-(4-hexylcyclohexyl)-ethan

Beispiel H

Man stellt ein Gemisch her aus:
7% 4-(5-Heptylpyrimidin-2-yl)-benzoesäure-(3-fluor-4-cyanphenyl)-ester
8% 2-(4-Cyanphenyl)-5-ethyl-1,3-dioxan
16% 2-(4-Cyanphenyl)-5-propyl-1,3-dioxan
18% 2-(4-Cyanphenyl)-5-butyl-1,3-dioxan
8% 4-Ethyl-4'-cyanbiphenyl
9% 4-Butyl-4'-cyanbiphenyl
6% 4-(4-Cyanphenyl)-4'-pentylbiphenyl
5% trans-4-(4-Ethylcyclohexyl)-benzoesäure-(4-cyanphenylester)
6% trans-4-(4-Pentylcyclohexyl)-benzoesäure-(4-cyanphenylester)
8% 4-Ethylbenzoesäure-3-(fluor-4-cyanphenyl-ester
9% 4-Propylbenzoesäure-(3-fluor-4-cyanphenyl-ester

Die Mischung weist die nachfolgenden physikalischen Daten auf: Klärpunkt 64°; Phasenübergangstemperatur smektisch/nematisch $<-20°$; Viskosität bei 20° 76 cP; $\Delta n = 0{,}175$; $\Delta\varepsilon = 33{,}2$.

**Patentansprüche**

1. Benzonitrile der Formel I,

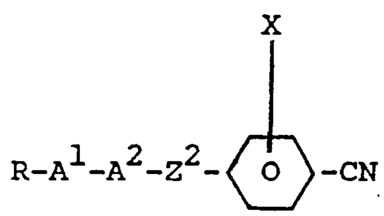

(I)

worin

R Alkyl mit 1—15 C-Atomen, worin auch eine oder zwei nicht benachbarte $CH_2$-Gruppen durch —O— ersetzt sein können, oder R'—$A^3$—$Z^3$—,

R' H, Alkyl mit 1—15 C-Atomen, worin auch eine oder zwei nicht benachbarte $CH_2$-Gruppen durch —O— ersetzt sein können,

$A^1$ Pyrimidin-2,5-diyl, Pyridin-2,5-diyl, Pyridazin-3,6-diyl oder 1,4-Phenylen,

$A^2$ Pyrimidin-2,5-diyl, Pyridin-2,5-diyl, 1,4-Phenylen oder 1,4-Cycohexylen,

$A^3$ Pyrimidin-2,5-diyl

$Z^2$ —CO—O—, —$CH_2CH_2$—, —$CH_2O$—,

$Z^3$ —CO—O—,

X F, Cl oder $CF_3$ bedeutet,

mit den Maßgaben, daß

a) mindestens einer der Ringe $A^1$, $A^2$, $A^3$ ein stickstoffhaltiger aromatischer Heterocyclus ist, und

b) trans-4-(5-Pentylpyrimidin-2-yl)-cyclohexylmethyl-(p-cyan-m-fluorphenyl)-ether,
trans-4-(5-Heptylpyrimidin-2-yl)-cyclohexylmethyl-(p-cyan-m-fluorphenyl)-ether,
trans-4-(5-Nonylpyrimidin-2-yl)-cyclohexylmethyl-(p-cyan-m-fluorphenyl)-ether,
trans-4-(5-Decylpyrimidin-2-yl)-cyclohexylmethyl-(p-cyan-m-fluorphenyl)-ether,
trans-4-(5-Dodecylpyrimidin-2-yl)-cyclohexylmethyl-(p-cyan-m-fluorphenyl)-ether,
p-(5-n-Heptylpyrimidin-2-yl)-benzyl-(4-cyan-3-fluorphenyl)-ether,
p-(5-Propylpyrimidin-2-yl)-benzyl-(4-cyan-3-fluorphenyl)-ether,
p-(5-Butylpyrimidin-2-yl)-benzyl-(4-cyan-3-fluorphenyl)-ether,
p-(5-Pentylpyrimidin-2-yl)-benzyl-(4-cyan-3-fluorphenyl)-ether,
p-(5-Hexylpyrimidin-2-yl)-benzyl-(4-cyan-3-fluorphenyl)-ether,
p-(5-Octylpyrimidin-2-yl)-benzyl-(4-cyan-3-fluorphenyl)-ether,
p-(5-Nonylpyrimidin-2-yl)-benzyl-(4-cyan-3-fluorphenyl)-ether,
p-(5-Decylpyrimidin-2-yl)-benzyl-(4-cyan-3-fluorphenyl)-ether und
p-(5-Dodecylpyrimidin-2-yl)-benzyl-(4-cyan-3-fluorphenyl)-ether,
ausgeschlossen sind.

2. Verfahren zur Herstellung von Benzonitrilen der Formel I nach Anspruch 1 dadurch gekennzeichnet, daß man eine Verbindung, die sonst der Formel I entspricht aber an Stelle von H-Atomen eine oder mehrere reduzierbare Gruppen und/oder C—C-Bindungen enthält, mit einem reduzierenden Mittel behandelt,

oder daß man zu Herstellung von Verbindungen der Formel I, worin X F oder Cl bedeutet, in einem entsprechenden Diazoniumsalz die Diazoniumgruppe durch F oder Cl ersetzt,

oder daß man zur Herstellung von Estern der Formel I (worin $Z^2$ und/oder $Z^3$ —CO—O— bedeuten und/oder R und/oder R' eine Carboxylgruppe enthalten) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von Nitrilen der Formel I ein entsprechendes Carbonsäureamid dehydratisiert oder ein entsprechendes Carbonsäurehalogenid mit Sulfamid umsetzt,

oder daß man zur Herstellung von Ethern der Formel I (worin R und/oder R' eine Alkoxygruppe bedeutet und/oder $Z^2$ eine —$CH_2O$—Gruppe ist) eine entsprechende Hydroxyverbindung verethert,

und/oder daß man gegebenenfalls eine Chlor- verbindung der Formel I mit einem Cyanid umsetzt.

3. Verwendung der Verbindungen der Formel I nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente eine Verbindung der Formel I nach Anspruch 1 ist.

5. Flüssigkristallanzeigeelement, dadurch gekennzeichnet, daß es eine flüssigkristalline Phase nach Anspruch 4 enthält.

6. Elektrooptisches Anzeigeelement nach Anspruch 5, dadurch gekennzeichnet, daß es als Dielektrikum eine flüssigkristalline Phase nach Anspruch 4 enthält.

**Revendications**

1. Benzonitriles de formule I.

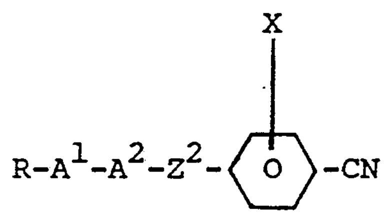

(I)

dans laquelle

R représente un groupe alkyl en C 1—C 15 dans lequel un ou deux groupes $CH_2$ non voisins peuvent être remplacés par —O—, ou R'—$A^3$—$Z^3$—,

R' représente H, un groupe alkyle en ce C 1—C 15 dans lequel un ou deux groupes $CH_2$ non voisins peuvent être remplacés par —O—,

$A^1$ représente un groupe pyrimidine-2,5-diyle, pyridine-2,5-diyle, pyridazine-3,6-diyle ou 1,4-phénylène,

$A^2$ représente un groupe pyrimidine-2,5-diyle, pyridine-2,5-diyle, 1,4-phénylène ou 1,4-cyclohexylène,

$A^3$ représente un groupe pyrimidine-2,5-diyle,

$Z^2$ représente —CO—O—, —$CH_2CH_2$—, —$CH_2O$—,

$Z^3$ représente —CO—O—,

X représente F, Cl ou $CF_3$,

sous réserve que

a) l'un au moins des cycles $A^1$, $A^2$, $A^3$ est un hétérocycle aromatique azoté, et

b) l'éther trans-4-(5-pentylpyrimidine-2-yl)-cyclohexylméthyl-(p-cyano-m-fluorophénylique),
l'éther trans-4-(5-heptylpyrimidine-2-yl)-cyclohexylméthyl-(p-cyano-m-fluorophénylique),
l'éther trans-4-(5-nonylpyrimidine-2-yl)-cyclohexylméthyl-(p-cyano-m-fluorophénylique),
l'éther trans-4-(5-décylpyrimidine-2-yl)-cyclohexylméthyl-(p-cyano-m-fluorophénylique),
l'éther trans-4-(5-dodécylpyrimidine-2-yl)-cyclohexylméthyl-(p-cyano-m-fluorophénylique),
l'éther p-(5-n-heptylpyrimidine-2-yl)-benzyl-(4-cyano-3-fluorophénylique),
l'éther p-(5-propylpyrimidine-2-yl)-benzyl-(4-cyano-3-fluorophénylique),
l'éther p-(5-butylpyrimidine-2-yl)-benzyl-(4-cyano-3-fluorophénylique),
l'éther p-(5-pentylpyrimidine-2-yl)-benzyl-(4-cyano-3-fluorophénylique),
l'éther p-(5-hexylpyrimidine-2-yl)-benzyl-(4-cyano-3-fluorophénylique),
l'éther p-(5-octylpyrimidine-2-yl)-benzyl-(4-cyano-3-fluorophénylique),
l'éther p-(5-nonylpyrimidine-2-yl)-benzyl-(4-cyano-3-fluorophénylique),
l'éther p-(5-décylpyrimidine-2-yl)-benzyl-(4-cyano-3-fluorophénylique),
l'éther p-(5-dodécylpyrimidine-2-yl)-benzyl-(4-cyano-3-fluorophénylique),
sont exclus.

2. Procédé de préparation des benzonitriles de formule I de la revendication 1, caractérisé en ce que l'on traite par un agent réducteur un composé répondant par ailleurs à la formule I mais qui contient, à la place d'atomes d'hydrogène, un ou plusieurs groupes réductibles et/ou liaisons C—C,

ou bien en ce que, pour préparer des composés de formule I dans laquelle X représente F ou Cl, on part d'un sel de diazonium correspondant et on remplace le groupe diazonium par F ou Cl,

ou bien en ce que, pour préparer des esters de formule I (dans laquelle $Z^2$ et/ou $Z^3$ représentent —CO—O— et/ou R et/ou R′ contiennent un groupe carboxyle), on fait réagir un acide carboxylique correspondant ou l'un de ses dérivés réactifs avec un alcool correspondant ou l'un de ses dérivés réactifs,

ou bien en ce que, pour préparer des nitriles de formule I, on déshydrate un carboxamide correspondant ou bien on fait réagir un halogénure d'acide carboxylique correspondant avec le sulfonamide,

ou bien en ce que, pour préparer des éthers de formule I (dans laquelle R et/ou R′ représentent un groupe alcoxy et/ou $Z^2$ représente un groupe —$CH_2O$—), on éthérifie un composé hydroxylé correspondant,

et/ou en ce que l'on fait réagir éventuellement un composé chloré de formule I avec un cyanure.

3. Utilisation des composés de formule I de la revendication 1 en tant que composants de phases à cristaux liquides.

4. Phase à cristaux liquides contenant au moins deux composants à cristaux liquides et caractérisé en ce que l'un au moins des composants est un composé de formule I de la revendication 1.

5. Elément d'affichage à cristaux liquides, caractérisé en ce qu'il contient une phase à cristaux liquides selon la revendication 4.

6. Elément d'affichage électro-optique selon la revendication 5, caractérisée en ce qu'il contient en tant que diélectrique une phase à cristaux liquides selon la revendication 4.

## Claims

1. Benzonitriles of the formula I

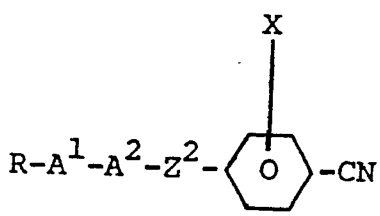

(I)

wherein

R is alkyl with 1—15 C atoms, it also being possible for one or two non-adjacent $CH_2$ groups to be replaced by —O—, or is $R'$—A—$^3$—$Z^3$,

R′ is H or alkyl with 1—15 C atoms, it also being possible for one or two non-adjacent $CH_2$ groups to be replaced by —O—,

$A^1$ is a pyrimidine-2,5-diyl group, a pyridine-2,5-diyl group, a pyridazine-3,6-diyl group or a 1,4-phenylene group,

$A^2$ is a pyrimidine-2,5-diyl group, a pyridine-2,5-diyl group, a 1,4-phenylene group or a 1,4-cyclohexylene group,

$A^3$ is a pyrimidine-2,5-diyl group,

$Z^2$ is —CO—O—, —$CH_2CH_2$— or —$CH_2O$—,

$Z^3$ is —CO—O—,

X is F, Cl or $CF_3$

with the proviso that

a) at least one of the rings $A^1$, $A^2$ or $A^3$ is a nitrogen-containing aromatic heterocyclic radical and

b) trans-4-(5-pentylpyrimidine-2-yl)-cyclohexylmethyl-(p-cyan-m-fluorphenyl)-ether,
trans-4-(5-heptylpyrimidine-2-yl)-cyclohexylmethyl-(p-cyan-m-fluorphenyl)-ether,
trans-4-(5-nonylpyrimidine-2-yl)-cyclohexylmethyl-(p-cyan-m-fluorphenyl)-ether,
trans-4-(5-decylpyrimidine-2-yl)-cyclohexylmethyl-(p-cyan-m-fluorphenyl)-ether,
trans-4-(5-dodecylpyrimidine-2-yl)-cyclohexylmethyl-(p-cyan-m-fluorphenyl)-ether,
p-(5-heptylpyrimidine-2-yl)-benzyl-(4-cyan-3-fluorphenyl)-ether,
p-(5-propylpyrimidine-2-yl)-benzyl-(4-cyan-3-fluorphenyl)-ether,
p-(5-butylpyrimidine-2-yl)-benzyl-(4-cyan-3-fluorphenyl)-ether,
p-(5-pentylpyrimidine-2-yl)-benzyl-(4-cyan-3-fluorphenyl)-ether,
p-(5-hexylpyrimidine-2-yl)-benzyl-(4-cyan-3-fluorphenyl)-ether,
p-(5-octylpyrimidine-2-yl)-benzyl-(4-cyan-3-fluorphenyl)-ether,
p-(5-nonylpyrimidine-2-yl)-benzyl-(4-cyan-3-fluorphenyl)-ether,
p-(5-decylpyrimidine-2-yl)-benzyl-(4-cyan-3-fluorphenyl)-ether and
p-(5-dodecylpyrimidine-2-yl)-benzyl-(4-cyan-3-fluorphenyl)-ether
are excluded.

2. Process for the preparation of benzonitriles of the formula I according to claim 1, characterized in that a compound which otherwise corresponds to the formula I but contains one or more reducible groups and/or C—C bonds instead of H atoms is treated with a reducing agent, or in that, to prepare compounds of

the formula I wherein X is F or Cl, the diazonium group in a corresponding diazonium salt is replaced by F or Cl, or in that, to prepare esters of the formula I (wherein $Z^2$ and/or $Z^3$ denote —CO—O— and/or R and/or R' contain a carboxyl group), a corresponding carboxylic acid or one of its reactive derivatives is reacted with a corresponding alcohol or one of its reactive derivatives, or in that, to prepare nitriles of the formula I, a corresponding carboxylic acid amide is dehydrated or a corresponding carboxylic acid halide is reacted with sulfamide, or in that, to prepare ethers of the formula I (wherein R and/or R' is an alkoxy group and/or $Z^2$ is —$CH_2O$ group), a corresponding hydroxy compound is etherified, and/or in that, if appropriate, a chlorine compound of the formula I is reacted with a cyanide.

3. Use of compounds of the formula I according to claim 1 as components of liquid crystal phases.

4. Liquid crystal phase with at least two liquid crystal components, characterized in that at least one component is a compound of the formula I according to claim 1.

5. Liquid crystal display element, characterized in that it contains a liquid crystal phase according to claim 4.

6. Electrooptical display element according to claim 5, characterized in that it contains a liquid crystal phase according to claim 4 as the dielectric.